# EUROPEAN PATENT SPECIFICATION

(11) **EP 3 837 698 B1**
(45) Date of publication and mention of the grant of the patent: **04.11.2026**
(21) Application number: 19762485.1
(22) Date of filing: 14.08.2019
(51) Int. Cl.: G16H 50/20, A61B 5/00

(54) **TISSUE PROPERTIES PRESENTATION SYSTEM**
SYSTEM ZUR PRÄSENTATION VON GEWEBEEIGENSCHAFTEN
SYSTÈME DE PRÉSENTATION DE PROPRIÉTÉS DE TISSUS

(30) Priority: 16.08.2018 US 201862764765 P
(43) Date of publication of application: 23.06.2021
(73) Proprietor: Dilon Medical Technologies Ltd., Caesarea 3088900 (IL)
(72) Inventor: COHEN, Gil, 9782224 Jerusalem (IL); AHARONOWITZ, Gal, 4493500 Sede Warburg (IL); GELTNER, Iddo, 4050000 Even Yehuda (IL); ZUR, Yoav, 3033115 Atlit (IL)
(74) Representative: Zacco GmbH
(86) International application number: PCT/IL2019/050909
(87) International publication number: WO 2020/035860

(56) References cited:
- WO-A1-2014/009893
- US-A1- 2011 264 000
- US-A1- 2013 231 548

## Description

### TECHNOLOGICAL FIELD

The invention is in the medical devices field and relates specifically to a system for displaying of measured tissue property(ies).

### BACKGROUND

Several medical examination procedures are currently used for characterizing tissue, e.g. for diagnosing certain disease/cancer. The doctor may order lab test(s), scan(s) or biopsy in order to characterize the tissue involved.

High or low levels of certain substances in the body can be a sign of disease/cancer. However, abnormal lab results are not a sure sign of disease/cancer. While being an important tool, doctors cannot rely on lab tests alone to diagnose disease/cancer.

Imaging procedures can help the doctor know whether an abnormal tissue (e.g. tumor) is present. These include for example, CT Scan, X-ray scan, Nuclear scan, Ultrasound and MRI.

In most cases, doctors need to do biopsy to be able to make diagnosis of the disease. In traditional biopsy procedures, the doctor removes one or more samples of suspected tissue under guidance of an imaging technique, such as Ultrasound. A pathologist then looks at the already extracted tissue under a microscope to characterize it. The tissue sample(s) may be removed in several ways such as by using a needle to withdraw tissue, or by performing a surgery.

Being an invasive procedure, biopsy should preferably be performed to withdraw as little as necessary tissue while still giving enough information about the extent of the disease/tumor inside the body.

WO2008072238**,** assigned to assignee of the present invention, describes a graphical user interface (GUI) including: (a) a group definition module adapted to accept a user input defining groups; (b) a data receiver operable to receive a plurality of individual measurement input datum indicative of status of a substrate; (c) a grouping module configured to assign each of the individual measurement input datum to one of the groups to produce grouped data; and (d) an output module adapted to output and present the grouped data to a user.

WO2014009893**,** also assigned to the assignee of the present invention, describes a graphical user interface configured for presenting information related to signals received from an array of sensors arranged in a predetermined spaced-apart relationship along a measurement axis, the presented information being in the form of an array of locations spaced-apart along an axis corresponding to the array of the sensors, and a corresponding array of measured data in association with the array of the locations, the array of measured data being indicative of signals received from the array of the sensors respectively, thereby providing an operator with information regarding a profile of a measured tissue profile along a tissue mass aligned with the array of sensors.

US 2013/231548 discloses a system for tissue analysis including sensors acquiring signals originating from a target tissue and representative of a tissue property. A signal converter converts the signals to property measurements and an analysis engine derives a variation of one tissue property with respect to another property and presents the variation in a graphical user interface.

### GENERAL DESCRIPTION

The present invention is defined by the appended claims. Subject-matter which does not fall under the scope of the claims is not part of the invention.

The present disclosure provides the doctor/medical staff with novel system and method (not claimed) for characterizing tissue being examined. The technique of the present disclosure assists the doctor/medical staff in on the spot decision making during tissue examination, e.g. during a biopsy procedure. The system of the present disclosure receives as an input online tissue sensing data indicative of one or more tissue properties, processes the sensing data and generates output data including one or more other tissue properties, and presents the output data to the doctor/medical staff in a graphical interface in a meaningful way, enabling the doctor/medical staff to decide about a course of action, such as a need for acquiring one or more tissue samples from a region under inspection in the body / tissue portion.

The system of the disclosure receives the tissue sensing data from one or more tissue characterization sensors connected to the system. The tissue characterization sensors measure and collect tissue sensing data indicative of one or more tissue properties, such as optical and/or acoustic and/or electrical tissue properties. In some embodiments, the tissue sensing data is indicative of electrical impedance of the tissue. Such tissue characterization sensors are described, for example, in US2003138378**,** US2012123296 and US2015305649, all assigned to the assignee of the present invention.

The system of the disclosure processes the input tissue sensing data and generates, as an output tissue data, two or more, specifically two, values of tissue characteristic parameters that are derived/calculated from the one or more measured tissue properties. The output tissue data, being two or above-two dimensional, is presented to a user in a suitable observation field on a graphical interface, enabling the user to make a decision about the tissue being measured. For example, if the user is using a connected biopsy device carrying the tissue characterization sensors, the user will be able to make an online decision relating to acquisition of a sample of the tissue portion being measured. The output tissue data can be presented graphically, numerically or in both fashions.

It is noted that the presentation of the values of tissue characteristic parameters is not necessarily arranged in the observation field in relation with or connection to the respective measured locations in the tissue and/or order of measurements being taken

If more than one tissue characterization sensor is used, the system is configured to distinguish between the different sensors, e.g. by allocating different graphical identifiers to the different sensors. The differentiation can be obtained graphically, for example, by color, and/or size, and/or shape of the displayed data.

The system is also configured to assist the user in the decision making by enabling the user to compare between the output tissue data and data representing known tissue types already/previously saved in the system. The system is configured with one or more saved tissue type maps, each tissue type map is constructed in the same parametric space as the output tissue data and identifies/defines one or more tissue types, such as fat tissue, types of malignant tissue, and the like.

The system is configured and operable to enable saving multiple arrays of the output tissue data measured and processed at different times (sequentially) during the measurement session, thereby saving history tissue sensing data, and to enable recalling by the user any one of the saved arrays. Saving the output tissue data can be automatic, or upon user input, or resulting from another action in the measurement session, e.g. if a biopsy device is used, the output tissue data can be saved automatically whenever the user operates the biopsy device to collect a tissue sample.

Thus, according to a broad aspect of the disclosure, there is provided a system configured and operable for presentation of tissue sensing data being collected, the system comprising a device having one or more hardware processors configured by machine-readable instructions to cause display of a graphical user interface on the device, the graphical user interface comprising at least one observation field and configured to be responsive to tissue sensing data, from a tissue being measured, comprising one or more sensing datum of measured tissue property, to transform each measured tissue property into virtual representation in said at least one observation field in the form of a parametric space of two different characteristic parameters of a tissue corresponding to the measured tissue property.

In some embodiments, the measured tissue property is electrical impedance and the two different tissue characteristic parameters include conductivity and permittivity values of the tissue being measured.

In some embodiments, the graphical user interface is configured and operable to be responsive to the tissue sensing data in real-time such that the virtual representation of the tissue sensing data is carried out in real-time in the at least one observation field.

In some embodiments, the graphical user interface is further configured and operable to be responsive to user input to present, in the at least one observation field, alone or together with the tissue sensing data, a user selected map of tissue types in the parametric space of the two different characteristic parameters of the tissue.

In some embodiments, the graphical user interface is configured and operable to be responsive to the tissue sensing data comprising a plurality of sensing datum measured from different locations in the tissue by a plurality of sensors and concurrently display the plurality of the sensing datum in the observation field.

In some embodiments, the system comprises a storage utility configured and operable for selectively storing the tissue sensing data based on a user input, an input from an external device connected to the system, or automatically. The graphical user interface may be configured and operable to be responsive to a user input to present, in the at least one observation field, at least some of the tissue sensing data stored in the storage utility, either alone or together with tissue sensing data being collected in real-time.

In some embodiments, the system comprises an indexing utility configured and operable to assign indexing data of one or more indices to each of the one or more sensing datum, the one or more indices being indicative of one or more measurement characteristics of each of the one or more sensing datum. The one or more measurement characteristics may include one or more of the following: time of measurement, origin of measurement, measurement session ID, acquisition of tissue sample being measured, and user defined tag. The storage utility may be configured and operable for selectively storing the tissue sensing data with the corresponding indexing data, based on a user input, an input from an external device connected thereto, or automatically.

In some embodiments, the graphical user interface is configured and operable to be responsive to a user input selecting one or more of the measurement characteristics, to present, in the at least one observation field, the tissue sensing data associated with at least the indexing data corresponding to the user selected measurement characteristic(s).

In some embodiments, the graphical user interface is configured and operable to be responsive to a user input selecting one of the measurement characteristics, to present, in the at least one observation field, all of the tissue sensing data associated with the selected measurement characteristic.

In some embodiments, the graphical user interface is configured and operable to be responsive to the one or more measurement characteristics and present the tissue sensing data with a distinguishing feature corresponding to each of the measurement characteristics. The distinguishing feature may include one or more of the following: shape, color and size of the virtual representation of the tissue sensing data. The graphical user interface may further comprise one or more additional observation fields for presenting to a viewer one or more of the following: the one or more distinguishing feature(s), numerical value of the two tissue characteristic parameters, and graphical representation of a connected external device.

In some embodiments, the graphical user interface is configured and operable to be responsive to value(s) of the measured tissue property of the tissue sensing data to dynamically define range of the parametric space presented in the observation field.

In some embodiments, the graphical user interface is configured and operable to be responsive to a user input selecting or defining one or more of the following:
- range of the parametric space,
- Partitioning of the parametric space into pre-defined regions,
- associating different colors or shapes or sizes of the virtual presentations with pre-defined values or value ranges of the measured tissue property or at least one of the two different tissue characteristic parameters, and
- presenting functions derived from the measured tissue property values.

According to another broad aspect of the disclosure, there is provided a method for presenting tissue sensing data being collected, the method comprising: receiving in real-time tissue sensing data comprising one or more sensing datum of measured tissue property value, transforming in real-time the measured tissue property value into a parametric space of two different characteristic parameters of a tissue, and virtually representing the parametric space in a corresponding observation field on a graphical user interface.

In some embodiments, the method further comprises receiving user input indicative of a user selected map of tissue types, and virtually presenting, in the observation field, the user selected map of tissue types in the parametric space of the two different characteristic parameters of the tissue.

In some embodiments, the method further comprises selectively storing the tissue sensing data based on at least one of the following: a user input, an input from an external device, or automatically. The method may further comprise presenting, in the observation field, at least some of the stored tissue sensing data, based on a user input.

In some embodiments, the method further comprises assigning indexing data of one or more indices to each of the one or more sensing datum, the one or more indices being indicative of one or more measurement characteristics of each of the one or more sensing datum. The one or more measurement characteristics may comprise one or more of the following: time of measurement, origin of measurement, measurement session ID, acquisition of tissue sample being measured, and user defined tag. The method may further comprise selectively storing the tissue sensing data with the corresponding indexing data, based on a user input, an input from an external device, or automatically. The method may further comprise receiving an input from a user for selecting one or more of the measurement characteristics, and presenting, in the observation field, the tissue sensing data associated with at least the indexing data corresponding to the user selected measurement characteristic(s). The method may further comprise receiving an input from a user for selecting one of the measurement characteristics, and presenting, in the observation field, all of the tissue sensing data associated with the selected measurement characteristic. The method may further comprise presenting the tissue sensing data with a distinguishing feature corresponding to each of the measurement characteristics. The distinguishing feature may be one or more of the following: shape, color and size of the virtual representation of the tissue sensing data. The method may comprise presenting, in one or more additional observation fields on the graphical user interface, one or more of the following: one or more distinguishing feature(s), numerical value of the two tissue characteristic parameters, and graphical representation of an external device providing the tissue sensing data.

In some embodiments, the method comprises dynamically defining range of the parametric space presented in the observation field based on value(s) of the measured tissue property of the tissue sensing data.

In some embodiments, the method further comprises receiving a user input selecting or defining one or more of the following:
- range of said parametric space,
- Partitioning of the parametric space into pre-defined regions,
- associating different colors or shapes or sizes of the virtual presentations with pre-defined values or value ranges of the measured tissue property or at least one of the two different tissue characteristic parameters, and
- presenting functions derived from the measured tissue property values.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig.** 1 illustrates by way of a block diagram an exemplary system according to a non-limiting embodiment of the present disclosure;
**Fig. 2** illustrates by way of a block diagram exemplary features of the system of the disclosure according to non-limiting embodiments of the present disclosure ; and
**Figs. 3A - 3G** exemplify a graphical user interface incorporating exemplary non-limiting features of the system of the present invention.

### DETAILED DESCRIPTION OF EMBODIMENTS

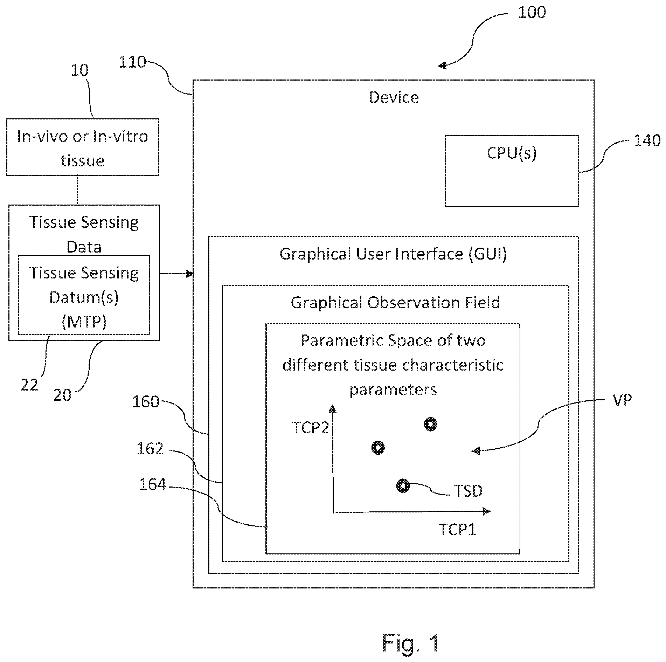

Reference is made to **Fig. 1** illustrating a non-limiting example of a system **100** configured in accordance with the present disclosure.

The system **100** is configured and operable for presenting tissue sensing data **20** being collected and includes a device **110** having one or more hardware processor units (CPU(s)) **140** configured to cause display of a graphical user interface **160** on the device. The graphical user interface **160** includes at least one observation field **162** and is configured to be responsive to the tissue sensing data **20** collected from the tissue **10** being measured in-vivo or in-vitro.

The tissue sensing data **20** includes one or more sensing datum(s) **22** of one or more measured tissue property **MTP** values. The device **110,** by its CPU(s) **140** is configured and operable to transform each measured tissue property **MTP** value (represented by one tissue sensing datum) into virtual representation **VP** in the at least one observation field **162** in the form of a parametric space **164** of two different tissue characteristic parameters, **TCP1** and **TCP2,** corresponding to the measured tissue property **MTP** value. In **Fig. 1****,** three tissue sensing datum **TSD** are displayed in the parametric space **164.**

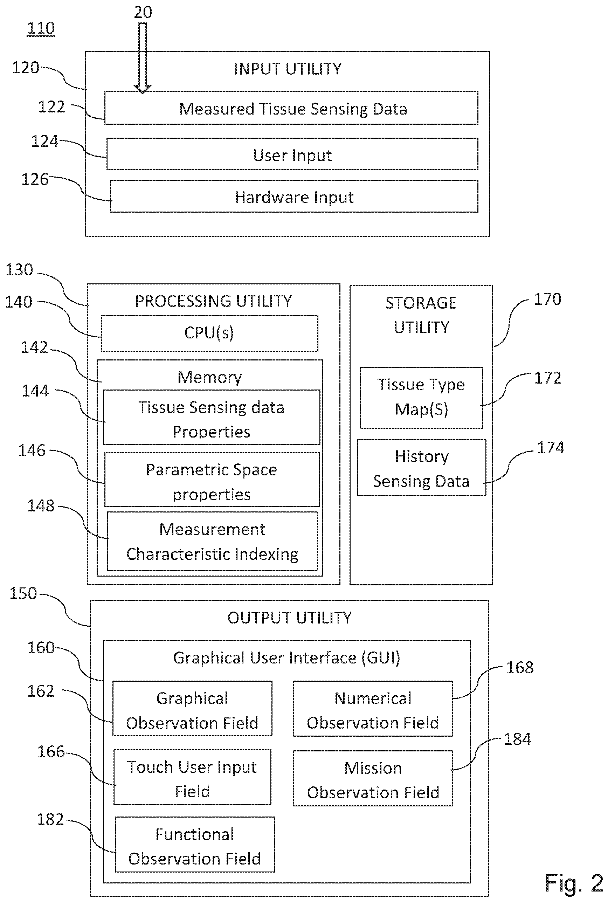

The device **110** can be a dedicated hardware loaded with a specific firmware, or can alternatively be a generic computing device running a specifically designed program (software). The device **110** includes a display (not shown) capable of presenting the GUI **160** to a user. Generally, as will be detailed further below in **Fig. 2****,** the device **110** includes an input utility configured for receiving input data including the tissue sensing data **20,** the processing unit(s) **140** and an output utility configured for presenting the GUI **160.** It is noted that, in some embodiments each of the utilities has its own CPU that controls its functionality, and in some other embodiments a single CPU may be connected to more than one utility/unit/module to control them. Therefore, the term CPU should be interpreted broadly, herein, meaning a controller/control unit and the like, and whenever a utility or a module is illustrated as configured to perform or be responsive to a certain action or process, it is equally meaning that the CPU controlling the specific utility or module is responsible for executing or being responsive to the mentioned action or process.

The device **110** can be configured, by the input utility and/or one or more of the CPU(s), to automatically identify at least a portion of the input data being the tissue sensing data **20.** Additionally, the device **110** can be configured, by the input utility and/or one or more of the CPU(s), to automatically identify connection of an external device, carrying one or more tissue characterization sensors, to the system **100,** to thereby receive therefrom sensing signals being indicative of the tissue sensing data **20.** As mentioned, the tissue sensing data **20** includes one or more tissue sensing datum **22,** each tissue sensing datum can be provided by a single sensor at a certain time. In case the tissue sensing data 20 includes a plurality of tissue sensing datum 22, they can be provided by a single sensor at a corresponding plurality of time points or by a plurality of sensors either simultaneously or sequentially at different times. The device 110 can also be configured to accept a user input via the input utility as will be further detailed below.

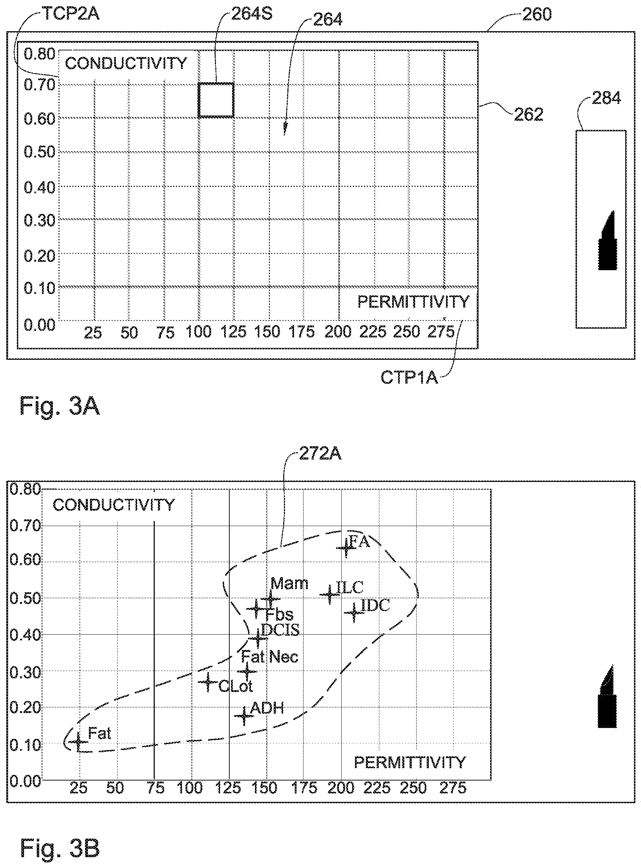

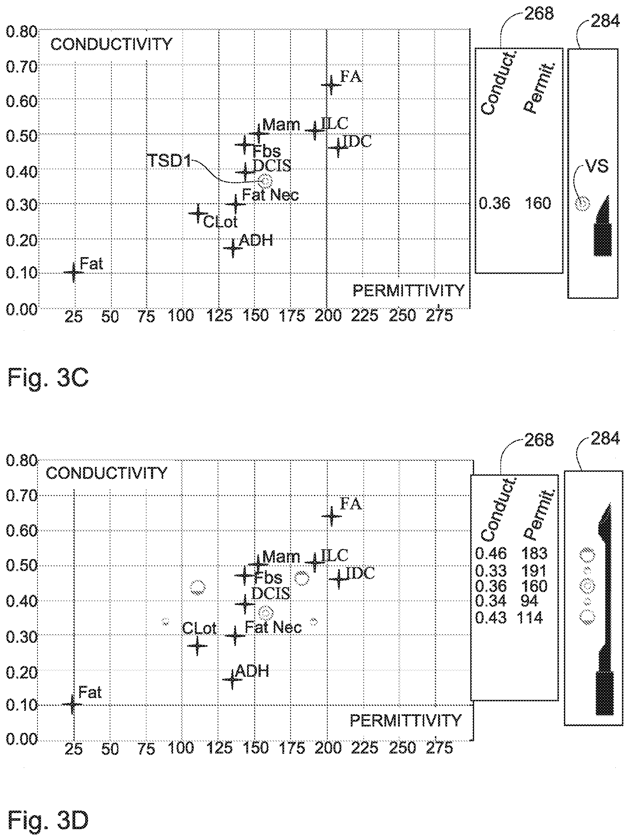

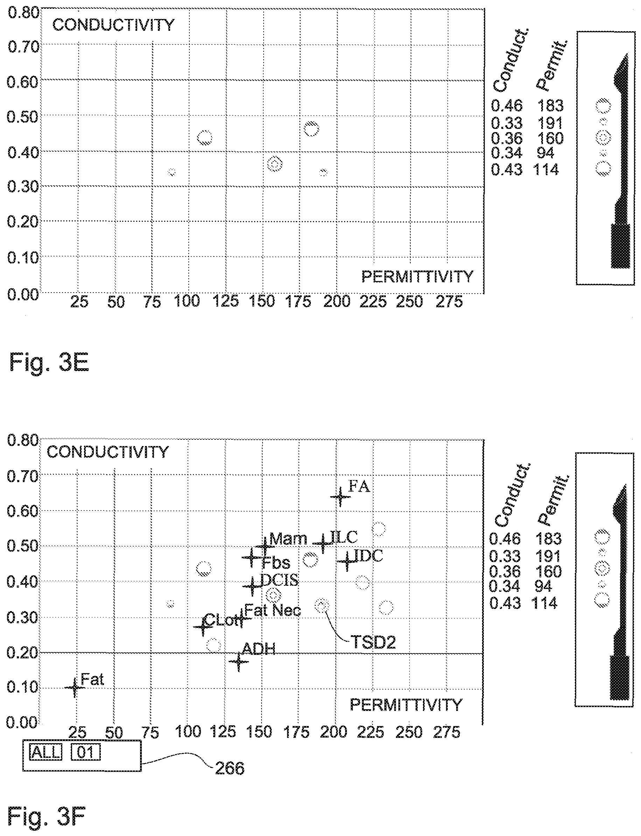

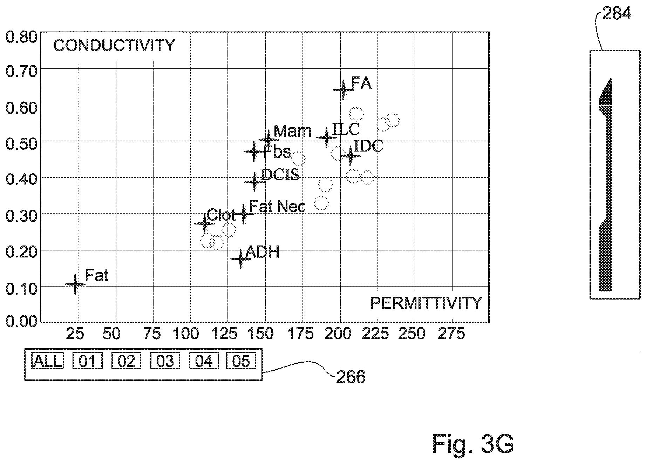

When tissue sensing data 20 is received at the device 110, the CPU(s) 140 process(es) the tissue sensing data 20, being indicative of one or more measured tissue properties MTP, and identify the tissue property(ies) being measured. In one non-limiting example, all the tissue sensing data is indicative of one measured tissue property. In another non-limiting example, the tissue sensing data is indicative of two or more measured tissue properties. As indicated above, the measured tissue property can be, for example, optical and/or acoustic and/or electrical property of the tissue. The CPU(s) **140** identifies each measured tissue property, typically received from a specific kind of tissue characterization sensor, and processes the relevant tissue sensing data in order to determine at least two different tissue characteristic parameters corresponding to the identified measured tissue property. For example, in case the measured tissue property is optical, the CPU(s) **140** is(are) configured to process the tissue optical data and determine, for example, two or more of the following: frequency, phase, intensity, absorbance, reflectance. In one specific non-limiting example, as will be further exemplified below with respect to **Fig. 3****,** the measured tissue property is electrical impedance and the at least two different tissue characteristic parameters, calculated and determined by the CPU(s) are the conductivity and permittivity. The device **110,** via the CPU(s) and/or the output utility (as described below), is configured for presenting one or more pairs of the determined tissue characteristic parameters, with a suitable (two-dimensional) parametric space, in the graphical observation field **162** of the graphical user interface **160,** for each tissue sensing datum **22** received. In case more than one measured tissue property is identified in the received tissue sensing data (e.g., optical and acoustical measured tissue properties), a corresponding number of graphical observation fields may be generated on the graphical user interface, each field presenting two calculated/derived tissue characteristic parameters corresponding to each one of the identifiable measured tissue properties.

Reference is now made to **Fig. 2** illustrating in more details different utilities, modules and sub-modules of the device **110,** some of them are optional, according to non-limiting embodiments of the present disclosure.

The device **110** generally includes an input utility **120,** a processing utility **130** and an output utility **150.** In addition, the device **110** includes a storage utility **170** for saving tissue sensing data or data indicative thereof and tissue type map(s), or can alternatively be configured with a network communication utility (not shown) enabling the device to access an external, remotely accessible, storage utility.

The input utility **120** includes a tissue sensing data module **122** configured and operable to accept as input the tissue sensing data **20** measured from a tissue portion (in-vivo or in-vitro), and a user input module **124** configured and operable to accept an input from a person using the device.

The tissue sensing data module **122** can be configured to automatically identify input data being the tissue sensing data **20.** Additionally, the tissue sensing data module **122** can be configured to automatically identify connection of an external device, carrying one or more tissue characterization sensors, to the system **100,** to thereby receive therefrom sensing signals being indicative of the tissue sensing data **20.** The tissue sensing data module **122** may also be configured to distinguish between a plurality of tissue sensing datum collected by different tissue characterization sensors. The tissue sensing data module **122** is connected to the processing utility **130** and configured and operable to transmit the tissue sensing data to the processing utility **140** for processing.

The user input module **124** can be configured to accept user input in a variety of ways. In one non-limiting example, the user can use a keyboard, a mouse or other similar devices to input data to the device **110.** In another non-limiting example, the user can use a touch interface to input data to the device **110,** the touch interface can be configured as a touch user input field **166** that forms part of or is embedded in the graphical user interface **160.** In yet another non-limiting example, the user can use audio interface (not specifically illustrated) to input data to the device **110.** The user input module **124** can be used to enter various user inputs relating to the information and data presented on the GUI **160,** as will be further detailed below.

The processing utility **130** includes the CPU(s) **140** that can be distributed in more than one location/utility (in case a plurality of CPUs exist in the device). Each CPU is configured and operable to process and execute the various actions needed to receive and process the measured tissue sensing data **20** in order to cause presentation of the processed (calculated/derived) at least two different tissue characteristic parameters in a suitable parametric space in the GUI **160.** The processing utility **130** also includes a memory **142,** in the form of a non-transitory and/or RAM memory, being configured and operable to save machine-readable instructions related to the processing and presentation of the tissue sensing data, as will be further described below.

The output utility **150** may be configured as or includes a display device configured for displaying the GUI **160** to the user. The GUI **160** includes one or more graphical observation fields **162** each configured, as described above, for displaying a parametric space of two different tissue characteristic parameters, for each measured tissue property received by the input utility **120.** Additionally, the GUI **160** may include one or more fields **166** for accepting user input by touch, and one or more numerical observation field(s) **168** configured for displaying numerical values of the at least two different tissue characteristic parameters, e.g. in a list form, such that each row displays the at least two different tissue characteristic parameters originating from a specific tissue characterization sensor.

The device **110** is generally configured for displaying the tissue sensing data on the GUI **160** in real-time, enabling real-time decision making such as deciding on tissue acquisition during a biopsy procedure. However, the device **110** can also be configured for saving the tissue sensing data, collected in each measurement session, for future reference and display, upon a user input, in an off-line mode. As such, the storage utility **170** includes a history sensing data module **174** configured for saving the received raw measured tissue sensing data, and/or the processed (calculated) at least two tissue characteristic parameters. The user can be then able to select, using a suitable user input, one or more of the saved tissue sensing data acquired during different time points in one or more measurement sessions, for display in the GUI. In some embodiments, the storage utility **170** is configured to be responsive to a user input to save one or more tissue sensing data being collected. In some embodiments, the input utility **120** includes a hardware input module **126** configured to identify and receive an input from a connected hardware, such as a biopsy device, and the storage utility **170** is configured to be responsive to the hardware input to save one or more tissue sensing data being collected. For example, during a biopsy procedure, collection of a tissue sample by a suitable movement of a part in the biopsy device can be identified by the hardware input module **126** as an act that initiates data saving and instructs the storage utility **170** (e.g. via a CPU **140** in the device) to save the tissue sensing data collected just prior to the tissue sample acquisition. Alternatively, the device **110** can be configured such that all the measured tissue sensing data, during a measurement session, is saved, automatically or upon a user input, in the storage utility **170.** The user can then access the saved data and selectively display the saved data, whether alone or in combination with real-time data, on the GUI **160.** The user can choose to display the data in various ways, as will be further described below, for example displaying some or all tissue sensing datum collected substantially concurrently (within a defined short time slot) by a plurality of tissue characterization sensors, or displaying some or all tissue sensing datum collected by the same tissue characterization sensor at different times.

The storage utility **170** may also include a tissue type map module **172** configured and operable to store one or more tissue type maps that can be displayed on the GUI **160** upon a user input or automatically based on the kind of the tissue sensing data being measured and/or the measurement session running. Each tissue type map includes one or more tissue types with their tissue characteristic parameters values matching the parametric space displayed on the GUI **160.** For example, if the parametric space displayed on the GUI **160** includes such tissue characteristic parameters as conductivity and permittivity, one saved tissue map can include one or more pairs of conductivity and permittivity values corresponding to respective one or more tissue types, such as conductivity and permittivity values for fat tissue, muscle tissue, etc. This way, by displaying the tissue type map on the GUI, alone or together with the real-time collected tissue data, the user can make on-line decisions relating to the tissue being measured, such as applying localized treatment to tissue and/or collecting one or more tissue samples for further examination in the lab. The user can select one map among a plurality of maps stored in the storage utility, or can on his own enter a map for display on the GUI.

The device **110** can also include, as part of the processing utility **130,** a measurement characteristic indexing module **148** configured and operable to tag each one of the measured tissue sensing datum with one or more tags (indices) indicative of one or more measurement characteristics. The tagging / indexing can be performed in real-time or at a subsequent stage. Generally, at least some of the tagging is done in real-time. Non-limiting examples of the measurement characteristics that can be assigned to each received tissue sensing datum include: time of measurement; origin of measurement (i.e. the tissue characterization sensor supplying the tissue sensing datum); tissue sampling act corresponding to the specific tissue sensing datum, i.e. whenever a tissue sample is collected by a biopsy device connected to the system **100** a suitable tag is assigned to the tissue sensing datum measured just before the tissue sampling. Additionally, the device may be configured to receive a user input defining a tag to be allocated to the tissue sensing datum. In one non-limiting example, the user can tag the received tissue sensing data by a name (ID) of the measurement session. The one or more indices assigned to the tissue sensing datum can be saved together with the tissue sensing datum, enabling the user to select and choose one or more measurement characteristics and to immediately or retroactively display, in the GUI **160,** the tissue sensing datum corresponding to the measurement characteristic(s) chosen.

The following are non-limiting examples of user selections of measurement characteristics: selecting a time slot in which all the tissue sensing data collected are displayed; selecting one or more tissue characterization sensors to display the tissue sensing data originating therefrom in a specific measurement session, during a defined time slot or in all the measurement sessions. The touch user input field **166** can be configured to enable the user selection of the measurement characteristics, e.g. measurement session IDs defined as numbers, tissue characterization sensors defined as numbers or logos or visual distinguishing features.

The processing utility **130** may also include machine-readable instructions enabling the user to apply, or which are applied automatically based on another input (whether from the user or from a connected hardware (e.g. via the hardware input module **126)),** to thereby manage the data presentation on the GUI **160.** In one non-limiting example, the properties of the parametric space, for each of the involved tissue characteristic parameters displayed, can be determined through a parametric space properties module **146.** For example, the range and/or the units and/or the presentation method (Cartesian, polar, etc.) can be determined and displayed automatically and dynamically (by the GUI) based on the received and presented tissue sensing data and/or calculated/derived tissue characteristic parameters, or can be determined beforehand or during the measurement session by the user. In another non-limiting example, the parametric space can be partitioned into pre-defined regions to assist in the data presentation and/or interpretation. The partitioning may be enhanced by the use of different colors for the different regions.

A tissue sensing data properties module **144** of the processing utility enables the user and/or the system (and the GUI) to assign a distinguishing feature for each tissue sensing datum presented on the GUI **160.** For example, different tissue sensing datum can be assigned different shapes and/or different colors and/or different sizes of the virtual presentation, to enable the user to distinguish between the different tissue sensing datum, whether displayed concurrently or sequentially on the GUI. For example, the shape and/or color and/or size of the virtual representation of each tissue sensing datum can be unique and different from the other tissue sensing datum, enabling the user to differentiate between the different tissue sensing datum presented. Non-limiting examples of using the distinguishing presentation features include: displaying different tissue sensing datum originating from respective different tissue characterization sensors in different shapes and/or different colors and/or different sizes. Specifically, only one of the shape or color or size distinguishing features can be changed to differentiate between the displayed tissue sensing datum. Furthermore, the tissue sensing data properties module **144** may also enable the user, via a suitable user input, to determine threshold value(s) of the displayed tissue sensing data such that tissue sensing datum value above or below the threshold value is presented on the GUI with a distinguishing feature being its shape, color or size. Additionally, the tissue sensing data properties module **144** may also enable the user, via a suitable user input, to select built-in or define functions of the measured and/or calculated/derived tissue characteristic parameters. The functions, being in equation or graph form, can then be displayed in a functional observation field **182** on the GUI **160.**

The tissue sensing data properties module **144** also enables the user, via a suitable user input, to determine which measured tissue properties or tissue characteristic parameters are to be displayed on the GUI, whether in real-time or retroactively in the off-line mode. For example, if the measured tissue property is the optical response, the user can decide to display any two or more of the following tissue characteristic parameters: optical absorbance, optical reflectance, optical transmission, and each of the aforementioned properties/characteristic parameters can be displayed in the intensity or frequency domains.

The GUI **160** may have additional observation fields responsive to actions relating to the tissue examination procedure. A mission observation field **184** on the GUI **160** may be used to display, either automatically or manually upon a user input, a graphical representation of an external tissue treatment device once connected to the device **110.** For example, the mission observation field **184** may display a graphical representation of a biopsy device with or without a tissue characterization sensor array mounted thereon. The tissue characterization sensors can be presented with the same distinguishing feature used to differentiate between the tissue sensing datum in the parametric space on the graphical observation field **162.**

Reference is now made to Figs. **3A-3G** illustrating a non-limiting example of a GUI with various observation fields according to some embodiments of the present invention. The present example illustrates some of the features of the device when used with a biopsy device carrying an array of tissue characterization sensors which are configured and operable for sensing the tissue electrical impedance property. To facilitate description of the various options of the exemplified GUI, features and elements similar to the above described features and elements of device **110** and GUI **160** will be given similar reference numbers with a difference of **100.** For example, the number **260** presents a GUI and the number **264** presents the parametric space displayed on the GUI **260.**

**Fig. 3A** illustrates a GUI **260** configured with one or more graphical observation field(s) **262** (one is shown) that include(s) parametric space **264** in the form of Cartesian coordinates of two calculated tissue characteristic parameters **TCP1A** and **TCP2A,** being permittivity (on the x-axis) and conductivity (on the y-axis). As described above, the TCP1A and TCP2A can be defined by the user or in some cases can be displayed automatically based on identification of the specific external device connected to the system. As shown, the two-dimensional parametric space **264** is also partitioned by grid lines (which is optional), defining squares **264S** used to visually assist a user in localization and/or interpretation of tissue sensing datum that will be displayed in the parametric space. Also shown is a mission observation field **284** that displays a graphical presentation of an external biopsy device connected to the system of the invention. The mission observation field **284** of the GUI **260** can be configured to reflect the actual state of the connected external device. In this instance, the GUI is shown at the start of a tissue biopsy examination procedure in which the device is still in its closed position. The graphical representation of the biopsy device can be dynamic and follows the different on-line states of the biopsy device during a measurement session, as will be further described below.

**Fig. 3B** illustrates the GUI **260** displaying, typically based on a user selection and input, or alternatively automatically by the processing utility **130** based on input data provided by the user or the connected hardware, a tissue type map **272A** which is usually stored in the storage utility **170.** As shown in this example, the displayed tissue type map includes a plurality of conductivity-permittivity value pairs corresponding to different tissue types within the breast. The conductivity-permittivity value pairs corresponding to the different tissue types are obtained, for example, from values reported in the literature, or from previous measurements performed and saved in the storage utility **170.** The conductivity-permittivity value pairs corresponding to different tissue types help the user while navigating with the biopsy device carrying the tissue characterization sensors to identify the examined tissue.

**Fig. 3C** illustrates the start of the tissue characterization procedure by the connected biopsy device. The biopsy device is still in its closed state and carries one tissue characterization sensor on its tip, a virtual sensor representation VS of the sensor's location on the biopsy device is shown beside the device representation in the mission observation field **284.** The tissue sensing datum TSD1 measured by the tissue characterization sensor is displayed on the parametric space **264** with a shape matching the shape of the virtual representation of the sensor VS. Also shown in the figure, is a numerical observation field **268** in which the values of the tissue characteristic parameters - the permittivity and the conductivity - of the TSD1 are displayed beside the virtual representation of the sensor VS. As the user navigates with the biopsy device inside the examined tissue, the values of the tissue characteristic parameters, the permittivity and conductivity, may change and the location of the TSSD1 in the parametric space changes accordingly.

**Fig. 3D** illustrates an open state of the biopsy device in which a plurality of tissue characterization sensors carried thereby are active and are virtually represented beside the device in the mission observation field **284.** In this specific example, the biopsy device includes five sensors, in addition to the tip sensor which is not shown now because it is not active in the open state of the biopsy device. As shown, each sensor is represented by a different shape to distinguish between them. Each sensor generates a TSD which is displayed in the parametric space together with the tissue type map. The numerical observation field also displays a list of five lines, where each line includes values of permittivity and conductivity and is displayed beside the respective sensor.

It should be noted that the GUI may be configured to adapt to the sensor configuration on the used biopsy device. For example, the biopsy device my not have a tissue characterization sensor on its tip. In this case, the GUI screen of **Fig. 3C** will not be part of the GUI In another example, there may be more, or less, sensors on the biopsy needle in the open configuration shown in **Fig. 3D****.**

**Fig. 3E** exemplifies how the user can activate or deactivate the display of the tissue type map together with the display of the on-line tissue sensing data.

**Fig. 3F** illustrates the feature of saving the tissue sensing data in the storage utility **170,** either automatically or manually by an input from the user, during a measurement session, and selectively displaying saved data together with on-line measured tissue sensing data. As shown, the already saved data can be displayed, upon a user selection and input via dedicated touch buttons, numbered with simple numbers indicating groups of saved data, in the touch user input field **266.** As shown, the online-data is displayed with each tissue sensing datum, originating from a different tissue characterization sensor, having a different shape, while all the previously collected and saved five tissue sensing datum are displayed with the same shape, for example a circle. As described above, each measured and saved tissue sensing datum is tagged with one or more measurement characteristics. In this example, the user chooses the biopsy taking event (tissue sampling act) indexing for displaying the saved data. In another example, the user may choose the time of measurement indexing for displaying the saved data. In other words, all the tissue sensing datum saved during a previous measurement session or during a time slot in the measurement session, are displayed. Therefore, all the saved data are presented with the same shape. If, however, in another example, the user chooses also the origin of measurement characteristic to be displayed, then the different saved tissue sensing datum may be displayed with different shapes to indicate the sensor by which the tissue sensing datum was collected. Additionally, the saved data and the online data can be displayed with different colors, to further facilitate user understanding of the displayed data. One of the uses for displaying saved data together with on-line data is to help a user come back to a specific measured location in the tissue in case the tissue location is medically important, and by this enable the user to treat or sample the tissue at that location. Such ability of the system is illustrated in the figure by the tissue sensing datum TSD2. As shown, a value of online datum from the second sensor (when the five sensors are counted from above downwards) coincides with a saved value of datum measured by one of the sensors. Another use for displaying saved data together with on-line data is to help a user assess whether specific types of tissue have been already sampled, in order to evaluate if there is a need to take additional biopsy samples from the current instantaneous (real-time) locations of the biopsy device.

**Fig. 3G** exemplifies the ability of a user to use the touch user input field **266** in order to select a plurality of saved data groups for concurrent display in the parametric space in the graphical observation field. This is an off-line mode/state in which no signals are arriving from any of the sensors which are inactive as illustrated in the mission observation field **284.** As shown, five groups of tissue sensing data, numbered **01** - **05,** have been saved already, and the user can select any of the groups or any combination thereof for display in the parametric space. In the shown example, the user selected the highlighted saved groups **01** and **03** for display.

It should be understood that the off-line mode can also be implemented without a biopsy device connected, or by using a hardware configuration (e.g. a computing device such as a tablet, desktop, laptop) with no connection to a biopsy device, or not being configured for receiving tissue sensing data. The saved data groups analyzed off-line are retrieved from the storage utility **170.**

Accordingly, the present technology provides a user with a comprehensive tool for on-line as well as off-line data display in ways that assist the user in decision making for tissue diagnosis and/or treatment.

The present invention is defined by the following claims.

## Claims

1. A system (100) for helping a user to make on-line decisions relating to acquiring one or more tissue samples from a region under inspection in a tissue being measured, configured and operable for presentation of tissue sensing data being collected, the system comprising a device (110) having one or more hardware processors (140) configured by machine-readable instructions to cause displaying of a graphical user interface (160) on the device (110), the graphical user interface (160) comprising at least one observation field (162) and configured to be responsive in real-time to tissue sensing data (20), from a tissue (10) being measured, comprising one or more sensing datum (22) of measured tissue property value, to transform, in real-time, each measured tissue property value into virtual real-time representation in said at least one observation field (162) in the form of a parametric space (164) of two different tissue characteristic parameters being calculated from said measured tissue property value; wherein said graphical user interface (160) is further configured and operable to be responsive to user input to present in real-time, in said at least one observation field (162), together with said tissue sensing data, a user selected map (172) of tissue types in said parametric space of the two different tissue characteristic parameters;
wherein said measured tissue property is electrical impedance, and said two different tissue characteristic parameters are conductivity and permittivity values of the tissue being measured.

2. The system (100) of any one of the preceding claims, wherein said graphical user interface (160) is configured and operable to be responsive to the tissue sensing data (20) comprising a plurality of sensing datum measured from different locations in the tissue by a plurality of sensors and concurrently display said plurality of the sensing datum in said observation field (162).

3. The system (100) of any one of the preceding claims, comprising a storage utility (170) configured and operable for selectively storing said tissue sensing data (20) based on a user input (124), an input from an external device (126) connected thereto, or automatically; wherein said graphical user interface (160) is configured and operable to be responsive to a user input to present, in said at least one observation field (162), at least some of the tissue sensing data stored in the storage utility, either alone or together with the tissue sensing data being collected in real-time.

4. The system (100) of claim 3, comprising an indexing utility configured and operable to assign indexing data of one or more indices to each of said one or more sensing datum (22), the one or more indices being indicative of one or more measurement characteristics of each of said one or more sensing datum; the one or more measurement characteristics comprise one or more of the following: time of measurement, origin of measurement, measurement session ID, acquisition of tissue sample being measured, and user defined tag; wherein said storage utility (170) is configured and operable for selectively storing said tissue sensing data (20) with the corresponding indexing data, based on a user input, an input from the external device connected thereto, or automatically; and wherein said graphical user interface (160) is configured and operable to be responsive to a user input selecting one or more of said measurement characteristics, to present, in said at least one observation field (162), at least said tissue sensing data associated with the indexing data corresponding to the user selected measurement characteristic(s).

5. The system (100) of claim 4, wherein said graphical user interface (160) is configured and operable to be responsive to said one or more measurement characteristics and present said tissue sensing data (20) with a distinguishing feature corresponding to each of said measurement characteristics, wherein said distinguishing feature comprises one or more of the following: shape, color and size of the virtual representation of the tissue sensing data.

6. The system (100) of claim 5, wherein said graphical user interface (160) further comprises one or more additional observation fields (162) for presenting to a viewer one or more of the following: said one or more distinguishing feature(s), numerical value of said two tissue characteristic parameters, and graphical representation of a connected external device.

7. The system (100) of any one of the preceding claims, wherein said graphical user interface (160) is configured and operable to be responsive to one or more of the following:
a) value(s) of said measured tissue property of said tissue sensing data (20) to dynamically define range of said parametric space presented in said observation field, and
b) a user input selecting or defining one or more of the following:
- range of said parametric space,
- Partitioning of the parametric space into pre-defined regions,
- associating different colors or shapes or sizes of the virtual presentations with pre-defined values or value ranges of the measured tissue property or at least one of the two different tissue characteristic parameters, and
- Presenting functions derived from the measured tissue property values.

## Patentansprüche

1. System (100) zur Unterstützung eines Benutzers bei Online-Entscheidungen in Bezug auf die Entnahme einer oder mehrerer Gewebeproben aus einer Region, die untersucht wird, in einem Gewebe, das gemessen wird, wobei das System zur Darstellung von Gewebeerfassungsdaten, die gesammelt werden, konfiguriert und betreibbar ist und eine Vorrichtung (110) mit einem oder mehreren Hardwareprozessoren (140) umfasst, die durch maschinenlesbare Anweisungen dazu konfiguriert sind, die Anzeige einer grafischen Benutzeroberfläche (160) auf der Vorrichtung (110) zu bewirken, wobei die grafische Benutzeroberfläche (160) mindestens ein Beobachtungsfeld (162) umfasst und dazu konfiguriert ist, in Echtzeit auf Gewebeerfassungsdaten (20) aus einem Gewebe (10), das gemessen wird, die ein oder mehrere Erfassungsdaten (22) eines Werts der gemessenen Gewebeeigenschaft umfassen, zu reagieren, um jeden Wert der gemessenen Gewebeeigenschaft in Echtzeit in eine virtuelle Echtzeitdarstellung in dem genannten mindestens einen Beobachtungsfeld (162) in Form eines Parameterraums (164) zweier unterschiedlicher charakteristischer Parameter des Gewebes umzuwandeln, die aus dem genannten Wert der gemessenen Gewebeeigenschaft berechnet werden; wobei die genannte grafische Benutzeroberfläche (160) ferner dazu konfiguriert und betreibbar ist, auf eine Benutzereingabe zu reagieren, um in Echtzeit in dem genannten mindestens einen Beobachtungsfeld (162) zusammen mit den genannten Gewebeerfassungsdaten eine vom Benutzer ausgewählte Karte (172) von Gewebetypen in dem genannten Parameterraum der zwei unterschiedlichen charakteristischen Parameter des Gewebes darzustellen;
wobei die genannte gemessene Gewebeeigenschaft die elektrische Impedanz ist und die genannten zwei unterschiedlichen charakteristischen Parameter des Gewebes Leitfähigkeits- und Permittivitätswerte des Gewebes, das gemessen wird.

2. System (100) nach einem der vorhergehenden Ansprüche, wobei die genannte grafische Benutzeroberfläche (160) dazu konfiguriert und betreibbar ist, auf die Gewebeerfassungsdaten (20), die eine Mehrzahl von Erfassungsdaten umfassen, die an unterschiedlichen Stellen im Gewebe von einer Mehrzahl von Sensoren gemessen werden, zu reagieren und die genannte Mehrzahl der Erfassungsdaten gleichzeitig in dem genannten Beobachtungsfeld (162) anzuzeigen.

3. System (100) nach einem der vorhergehenden Ansprüche, umfassend eine Speichereinrichtung (170), die dazu konfiguriert und betreibbar ist, die genannten Gewebeerfassungsdaten (20) selektiv auf Grundlage einer Benutzereingabe (124), einer Eingabe von einer damit verbundenen externen Vorrichtung (126) oder automatisch zu speichern; wobei die genannte grafische Benutzeroberfläche (160) dazu konfiguriert und betreibbar ist, auf eine Benutzereingabe zu reagieren, um in dem genannten mindestens einen Beobachtungsfeld (162) zumindest einige der in der Speichereinrichtung gespeicherten Gewebeerfassungsdaten darzustellen, entweder allein oder zusammen mit den Gewebeerfassungsdaten, die in Echtzeit gesammelt werden.

4. System (100) nach Anspruch 3, umfassend eine Indexierungseinrichtung, die dazu konfiguriert und betreibbar ist, jedem der genannten einen oder mehreren Erfassungsdaten (22) Indexierungsdaten eines oder mehrerer Indizes zuzuordnen, wobei der eine oder die mehreren Indizes für ein oder mehrere Messmerkmale jedes der genannten einen oder mehreren Erfassungsdaten indikativ sind; das eine oder die mehreren Messmerkmale umfassen eines oder mehrere der Folgenden: Zeitpunkt der Messung, Ursprung der Messung, Messsitzungs-ID, Entnahme der Gewebeprobe, die gemessen wird, und benutzerdefinierte Markierung; wobei die genannte Speichereinrichtung (170) dazu konfiguriert und betreibbar ist, die genannten Gewebeerfassungsdaten (20) mit den entsprechenden Indexierungsdaten selektiv auf Grundlage einer Benutzereingabe, einer Eingabe von der damit verbundenen externen Vorrichtung oder automatisch zu speichern; und wobei die genannte grafische Benutzeroberfläche (160) dazu konfiguriert und betreibbar ist, auf eine Benutzereingabe, die eines oder mehrere der genannten Messmerkmale auswählt, zu reagieren, um in dem genannten mindestens einen Beobachtungsfeld (162) zumindest die genannten Gewebeerfassungsdaten darzustellen, die den Indexierungsdaten zugeordnet sind, die dem/den vom Benutzer ausgewählten Messmerkmal(en) entsprechen.

5. System (100) nach Anspruch 4, wobei die genannte grafische Benutzeroberfläche (160) dazu konfiguriert und betreibbar ist, auf die genannten einen oder mehreren Messmerkmale zu reagieren und die genannten Gewebeerfassungsdaten (20) mit einem Unterscheidungsmerkmal darzustellen, das jedem der genannten Messmerkmale entspricht, wobei das genannte Unterscheidungsmerkmal eines oder mehrere der Folgenden umfasst: Form, Farbe und Größe der virtuellen Darstellung der Gewebeerfassungsdaten.

6. System (100) nach Anspruch 5, wobei die genannte grafische Benutzeroberfläche (160) ferner ein oder mehrere zusätzliche Beobachtungsfelder (162) umfasst, um einem Betrachter eines oder mehrere der Folgenden darzustellen: die genannten einen oder mehreren Unterscheidungsmerkmal(e), den Zahlenwert der genannten zwei charakteristischen Parameter des Gewebes und eine grafische Darstellung einer angeschlossenen externen Vorrichtung.

7. System (100) nach einem der vorhergehenden Ansprüche, wobei die genannte grafische Benutzeroberfläche (160) dazu konfiguriert und betreibbar ist, auf eines oder mehrere der Folgenden zu reagieren:
a) Wert(e) der genannten gemessenen Gewebeeigenschaft der genannten Gewebeerfassungsdaten (20), um den Bereich des genannten Parameterraums, der in dem genannten Beobachtungsfeld dargestellt wird, dynamisch festzulegen, und
b) eine Benutzereingabe, die eines oder mehrere der Folgenden auswählt oder festlegt:
- Bereich des genannten Parameterraums,
- Aufteilung des Parameterraums in vordefinierte Regionen,
- Zuordnung unterschiedlicher Farben oder Formen oder Größen der virtuellen Darstellungen zu vordefinierten Werten oder Wertebereichen der gemessenen Gewebeeigenschaft oder mindestens eines der zwei unterschiedlichen charakteristischen Parameter des Gewebes, und
- Darstellung von Funktionen, die aus den Werten der gemessenen Gewebeeigenschaft abgeleitet sind.

## Revendications

1. Système (100) destiné à aider un utilisateur à prendre des décisions en ligne relatives au prélèvement d'un ou plusieurs échantillons de tissu à partir d'une région qui est inspectée dans un tissu qui est mesuré, configuré et opérationnel pour la présentation de données de détection de tissu qui sont collectées, le système comprenant un dispositif (110) ayant un ou plusieurs processeurs matériels (140) configurés par des instructions lisibles par machine pour causer l'affichage d'une interface utilisateur graphique (160) sur le dispositif (110), l'interface utilisateur graphique (160) comprenant au moins un champ d'observation (162) et étant configurée pour réagir en temps réel à des données de détection de tissu (20), provenant d'un tissu (10) qui est mesuré, comprenant une ou plusieurs données de détection (22) de valeur de la propriété de tissu mesurée, pour transformer, en temps réel, chaque valeur de la propriété de tissu mesurée en une représentation virtuelle en temps réel dans ledit au moins un champ d'observation (162) sous la forme d'un espace paramétrique (164) de deux paramètres caractéristiques du tissu différents calculés à partir de ladite valeur de la propriété de tissu mesurée; dans lequel ladite interface utilisateur graphique (160) est en outre configurée et opérationnelle pour réagir à une entrée d'utilisateur pour présenter en temps réel, dans ledit au moins un champ d'observation (162), conjointement avec lesdites données de détection de tissu, une carte (172) de types de tissu sélectionnée par l'utilisateur dans ledit espace paramétrique des deux paramètres caractéristiques du tissu différents;
dans lequel ladite propriété de tissu mesurée est l'impédance électrique, et lesdits deux paramètres caractéristiques du tissu différents sont des valeurs de conductivité et de permittivité du tissu qui est mesuré.

2. Système (100) selon l'une quelconque des revendications précédentes, dans lequel ladite interface utilisateur graphique (160) est configurée et opérationnelle pour réagir aux données de détection de tissu (20) comprenant une pluralité de données de détection mesurées à partir de différents emplacements dans le tissu par une pluralité de capteurs et afficher simultanément ladite pluralité des données de détection dans ledit champ d'observation (162).

3. Système (100) selon l'une quelconque des revendications précédentes, comprenant une unité de stockage (170) configurée et opérationnelle pour stocker sélectivement lesdites données de détection de tissu (20) sur la base d'une entrée d'utilisateur (124), d'une entrée provenant d'un dispositif externe (126) qui y est connecté, ou automatiquement; dans lequel ladite interface utilisateur graphique (160) est configurée et opérationnelle pour réagir à une entrée d'utilisateur pour présenter, dans ledit au moins un champ d'observation (162), au moins certaines des données de détection de tissu stockées dans l'unité de stockage, soit seules, soit conjointement avec les données de détection de tissu qui sont collectées en temps réel.

4. Système (100) selon la revendication 3, comprenant une unité d'indexation configurée et opérationnelle pour attribuer des données d'indexation d'un ou plusieurs indices à chacune desdites une ou plusieurs données de détection (22), le ou les plusieurs indices étant indicatifs d'une ou plusieurs caractéristiques de mesure de chacune desdites une ou plusieurs données de détection; la ou les plusieurs caractéristiques de mesure comprennent une ou plusieurs parmi les suivantes: moment de la mesure, origine de la mesure, identifiant de session de mesure, prélèvement de l'échantillon de tissu qui est mesuré, et marqueur défini par l'utilisateur; dans lequel ladite unité de stockage (170) est configurée et opérationnelle pour stocker sélectivement lesdites données de détection de tissu (20) avec les données d'indexation correspondantes, sur la base d'une entrée d'utilisateur, d'une entrée provenant du dispositif externe qui y est connecté, ou automatiquement; et dans lequel ladite interface utilisateur graphique (160) est configurée et opérationnelle pour réagir à une entrée d'utilisateur sélectionnant une ou plusieurs desdites caractéristiques de mesure, pour présenter, dans ledit au moins un champ d'observation (162), au moins lesdites données de détection de tissu associées aux données d'indexation correspondant à la ou aux caractéristique(s) de mesure sélectionnées par l'utilisateur.

5. Système (100) selon la revendication 4, dans lequel ladite interface utilisateur graphique (160) est configurée et opérationnelle pour réagir auxdites une ou plusieurs caractéristiques de mesure et présenter lesdites données de détection de tissu (20) avec un élément distinctif correspondant à chacune desdites caractéristiques de mesure, dans lequel ledit élément distinctif comprend un ou plusieurs parmi les suivants: forme, couleur et taille de la représentation virtuelle des données de détection de tissu.

6. Système (100) selon la revendication 5, dans lequel ladite interface utilisateur graphique (160) comprend en outre un ou plusieurs champs d'observation (162) supplémentaires pour présenter à un observateur un ou plusieurs parmi les suivants: lesdits un ou plusieurs élément(s) distinctifs, valeur numérique desdits deux paramètres caractéristiques du tissu, et représentation graphique d'un dispositif externe connecté.

7. Système (100) selon l'une quelconque des revendications précédentes, dans lequel ladite interface utilisateur graphique (160) est configurée et opérationnelle pour réagir à un ou plusieurs parmi les suivants:
a) valeur(s) de ladite propriété de tissu mesurée desdites données de détection de tissu (20) pour définir de manière dynamique la plage dudit espace paramétrique présenté dans ledit champ d'observation, et
b) une entrée d'utilisateur sélectionnant ou définissant un ou plusieurs parmi les suivants:
- plage dudit espace paramétrique,
- partitionnement de l'espace paramétrique en régions prédéfinies,
- association de différentes couleurs ou formes ou tailles des présentations virtuelles à des valeurs prédéfinies ou des plages de valeurs de la propriété de tissu mesurée ou d'au moins l'un des deux paramètres caractéristiques du tissu différents, et
- présentation de fonctions dérivées des valeurs de la propriété de tissu mesurée.
